# EUROPEAN PATENT SPECIFICATION

(11) **EP 2 793 983 B1**
(45) Date of publication and mention of the grant of the patent: **13.06.2018**
(21) Application number: 12801601.1
(22) Date of filing: 14.12.2012
(51) Int. Cl.: A61M 15/00

(54) **ARRANGEMENT FOR A DRUG DELIVERY DEVICE AND METHOD OF ASSEMBLING THE SAME**
ANORDNUNG FÜR EINE ARZNEIMITTELABGABEVORRICHTUNG UND VERFAHREN ZU DEREN AUFBAU
AGENCEMENT POUR UN DISPOSITIF D'ADMINISTRATION DE MÉDICAMENT ET PROCÉDÉ D'ASSEMBLAGE DE CELUI-CI

(30) Priority: 21.12.2011 EP 11194966
(43) Date of publication of application: 29.10.2014
(73) Proprietor: Sanofi SA, 1214 Vernier (CH)
(72) Inventor: MAYER, Stefan, 79098 Freiburg im Breisgau (DE)
(74) Representative: Zwicker, Jörk
(86) International application number: PCT/EP2012/075623
(87) International publication number: WO 2013/092437

(56) References cited:
- EP-A1- 2 228 086
- WO-A1-2004/033009
- WO-A1-2010/076302
- US-A1- 2004 164 186
- US-A1- 2006 016 833
- US-A1- 2008 210 228

## Description

The present disclosure relates to an arrangement for a drug delivery device, e.g. an inhaler, such as a dry powder inhaler, and a method for assembling the same.

An inhaler is known from WO 2009/065708 A2 and EP 2 228 086 A1, for example.

It is an object of the present disclosure to provide one or more components which facilitate assembling of a drug delivery device and to provide an associated method for assembling a drug delivery device.

This object is achieved by the subject matter of the independent claims. Advantageous embodiments and refinements are subject matter of the dependent claims.

One aspect of the present invention relates to an inhaler according to claim 1. Another aspect of the present invention relates to a method of assembling an inhaler according to claim 11. Yet another aspect of the present invention relates to a kit according to claim 14.

One aspect of the present disclosure relates to an arrangement or system for a drug delivery device, such as an inhaler. The arrangement comprises a support and a connection part. The arrangement may further comprise a spring element, wherein the spring element may be arranged between the support and the connection part. The support comprises a support connection element and the connection part comprises a part connection element. The support connection element and the part connection element are configured to establish a releasable connection to releasably connect the support and the connection part.

An advantage of the present disclosure of the proposed arrangement is that it enables to form a unit from single components like the support and the connection part as well as optionally the spring element. The pre-assembled unit can be handled more easily than the single components as the support and the connection part may be firmly, but releasably, connected to one another. In particular, the spring element may be retained, if appropriate in a biased state, between the support and the connection part. Thus, the spring element may be kept in a reliably biased, e.g. compressed, state and can nevertheless be handled easily as it is retained between connection part and support which are connected to each other. Accordingly, a pre-assembled unit may be formed for the drug delivery device from the recited components of the arrangement when the releasable connection is established. During assembling of the drug delivery device, the releasable connection may be released and the connection part may be moved with respect to the support, for example by means of the spring force of the spring element which may now reduce its bias.

The support may support the spring element. The connection part may form a counter bearing for the spring element. Accordingly, the spring element may be arranged and retained between the support and the connection part.

In an embodiment, a unit is provided, which comprises the arrangement, wherein the releasable connection between the support and the connection part is established.

An aspect of the present disclosure relates to a module. The module may be connectable to the unit recited above, in particular to form the drug delivery device. The module may comprise one or more components to form the drug delivery device together with the components of the arrangement recited further above. For example, the module may comprise a reservoir configured to retain the drug, particularly a plurality of doses thereof, to be dispensed from the device, a dosing mechanism to prepare a dose of drug for dispense, and/or a body to retain further components of the module, etc. The module may comprise at least one release element or more than one release element. The release element may be configured to cooperate with at least one of the support connection element and the part connection element to release the releasable connection when the unit and the module are connected to one another. The release elements may be uniformly distributed in the module. The provision of a release element provides the advantage that the releasable connection can be easily released, without any separate or even external means, just during the regular movement necessary for assembling the unit and the module for the device. Once assembled, the unit and the module may be firmly, preferably irreleasably, connected to each other.

In an advantageous embodiment, the reservoir is arranged at an open end of the module. The reservoir may be filled with drug via an opening which faces the open end of the module. The open end and, particularly, the reservoir may be configured to receive the connection part. The support may be configured to be, e.g. permanently connected to the body. The support may form an outer surface of the device, e.g. the bottom surface.

An embodiment relates to a kit which comprises the unit and the module as described further above.

An embodiment relates to a drug delivery device which comprises the arrangement. The device may comprise the module recited above, which is connected to the arrangement. In the device, the releasable connection between the support and the connection part is not established. Accordingly, the connection part is movable relative to the support. The spring element may be arranged to move the connection part away from the support when the drug delivery device is operated to dispense a dose of the drug. This provides the advantage that the drug can be kept under pressure by the connection part. This facilitates provision of reproducible doses, i.e. a constant dose volume of the doses of drug which are dispensed from the reservoir, over the lifetime of the inhaler. The connection part may form a movable sealing base of the reservoir.

A further aspect of the present disclosure relates to a method of assembling a drug delivery device. Therein, a unit and a module, e.g. as recited further above, are provided. The unit comprises a support and a connection part which are releasably connected to each other. The module comprises a reservoir and an opening communicating with the reservoir. Additionally, the method comprises the step of filling drug into the reservoir via the opening. Thereafter, the unit and the module are connected with each other. The opening of the filled reservoir is closed with the connection part and the releasable connection is released in such a way that the connection part is movable with respect to the support. Closing the opening and the release of the releasable connection may take place simultaneously with the connection between the unit and the module or before the unit is connected to the module, e.g. before a movement between the module and the unit for connecting the unit and the module has been completed. Closing the opening may be enabled by the release of the releasable connection which may render the part connection element movable. Alternatively, the opening may already be closed, before the releasable connection is released, e.g. by moving the connection part into the reservoir before the releasable connection is released. During connection of the module and the unit, the support may be, e.g. firmly and/or irreleasably, connected to a body of the module, within which the reservoir may be formed or retained.

As an advantage, the method allows for easy assembling of a drug delivery device comprising a plurality of mechanically engaged and interacting parts by means of pre-assembled units or modules each, in turn, comprising a plurality of parts itself. Thereby, the unit and the module can be connected with the effect of the releasable connection being released and the connection part being accommodated movably with respect to the support within the reservoir.

In an embodiment, the support comprises a guide feature associated with and, particularly angularly, offset from the support connection element, wherein, when the releasable connection is established, the part connection element is, at least partly, arranged between the support connection element and the guide feature. This geometry may prevent a relative rotation of the support and the connection part, when said parts are releasably connected. Thus, the guide feature may stabilize the unit. Furthermore, the guide feature may guide the relative movement of the connection part and the support when they are connected to form the unit.

In an advantageous embodiment, the support forms the base of the drug delivery device and is arranged at the bottom end.

In an embodiment, the releasable connection is a snap-fit connection. The snap-fit connection is expediently formed by the connection elements. Accordingly, the connection elements may be designed as snap elements or snap means. Each support connection element may mate with a part connection element. The advantage of the configuration as snap-fit connection is the reliable mechanical engagement, whereby the releasable connection can be easily established and released.

In an embodiment, the arrangement comprises a desiccant which may be provided within a desiccator, e.g. a desiccator capsule. The desiccant may be arranged within an interior space delimited by the spring element. This provides for a particular space saving arrangement. The desiccant may avoid moisture induced by agglutination of drug in the reservoir of the device.

In an embodiment, the support connection element and the part connection element are further oriented along a respective axis, preferably along a common axis. The axis may be the longitudinal axis of the drug delivery device. The orientation of the connection elements along the common axis may define the direction along which the module and the unit have to be moved to connect the module and the unit and/or to release the releasable connection, after the unit and the module have been arranged such that the axes of the connection elements have been aligned.

In an advantageous embodiment one of the connection elements may be implemented flexible. In other words, one of the connection elements may be flexible. The other one of the connection elements may be rigid. This enables a simple displacement of the flexible connection element with respect to the other connection element in order to engage or disengage the connection elements and, consequently, to establish or to release the releasable connection, respectively.

In an embodiment, the connection part comprises a main body. The part connection element may comprise an axially oriented bar which is spaced apart from and connected to the main body of the connection part. As an advantage, this geometry may allow for a mechanical stabilization of the releasable connection against external torques, e.g., being applied to the connection part. Furthermore, the bar may provide a guide track for the release element during the connection of the unit and the module. Furthermore, the bar may provide a means for a visual filling level indicator of the device.

In an embodiment, the connection part comprises a seal member. The seal member may be configured to cooperate with a wall of the reservoir, e.g. to seal the reservoir, particularly the opening thereof. The seal member may be configured to form a drug-tight seal in cooperation with the wall of the reservoir. In an advantageous embodiment, the seal member is arranged at the top end of the connection part. The seal member may form the movable sealing base of the reservoir.

In an embodiment, particularly when the releasable connection is established, the flexible one of the support connection element and the part connection element has or provides an exposed surface. The exposed surface may be an oblique, preferably not perpendicular, surface. The exposed surface may have a surface normal defining an acute angle with the axis. The exposed surface may be a plane surface. The exposed surface may be provided by the flexible one of the part connection element and the support connection element. The exposed oblique surface may provide an interaction surface for cooperating with the release element to release the releasable connection.

In an embodiment, the support connection element and the part connection element are arranged and configured such that, when the releasable connection is not established, and the distance between the support connection element and the part connection element is decreased by a relative movement between the support and the connection part, the support connection element and the part connection element engage one another, thereby releasably connecting the support and the connection part. The spring element may be biased during this movement. Establishing and/or releasing the releasable connection may involve flexing the flexible one of the connection elements with respect to the other one of the connection elements.

In an embodiment, the releasable connection, when established, prevents relative movement between the support connection element and the part connection element, whereas the movement would increase the distance between the support connection element and the part connection element.

In an embodiment, when the releasable connection is established, the spring element is biased and disengagement of the support connection element and the part connection element is prevented by the spring force of the biased spring element which keeps the support connection element and the part connection element in abutment. Accordingly, the advantage is the biasing of the spring element and the fixation of the connection part relative to the support by the releasable connection.

In an embodiment, the assembling is carried out by moving the unit and the module axially towards one another, the release element thereby cooperating with the exposed surface of one of the support connection element and the part connection element, e.g. the flexible one of the connection elements. Further movement causes a deflection of the flexible one of the support connection element and the part connection element, thereby releasing the releasable connection, e.g. by disengaging the connection elements.

In an embodiment, the release of the releasable connection triggers a partly release of the biased spring element. The restoring force of the spring element may push the seal member of the connection part against the drug in the reservoir.

In an embodiment, after the releasable connection has been released, the flexible connection element may remain deflected, e.g. due to the release element still acting on the connection element. The deflected connection element may remain connected to the connection part or the support respectively, i.e. it may not be detached, e.g. broken, due to the deflection force acting on the element. Accordingly, there are no loose parts in the device once it was assembled although one of the connection elements may have been considerably deflected during connection of the module and the unit. Loose components in a device may, for example, disturb the visual indication of the filling level.

In an embodiment, one of the connection elements, such as the flexible one of the connection elements, e.g. the support connection element, is implemented hook-like. In other words the respective connection element may comprise a shaft and a projection projecting from the shaft. Provision of a flexible connection element is facilitated in this way. The part connection element may comprise a receiving section in order to receive the projection of the support connection element to establish the releasable connection.

In an embodiment, the support comprises a plurality of support connection elements.

In an embodiment, the connection part comprises a plurality of part connection elements. The part connection elements may, in each case, be configured to mate with a respective one of the support connection elements.

The support connection elements and/or the part connection elements may be uniformly distributed in the circumferential direction on the support or the connection part, respectively. A respective guide feature may be associated with each support connection element.

In an embodiment, a plurality of guide features and/or release elements may be provided. Expediently, part connection elements, support connection elements, guide features, and/or release elements are provided in equal numbers. The respective elements or features are expediently uniformly distributed in the circumferential direction.

Features which are described herein above and below in conjunction with different aspects or embodiments may also apply for other aspects and embodiments. Particularly, features described with respect to the arrangement may apply for the method, the unit and the module and vice versa.

Further features and advantages of the subject matter of this disclosure will become apparent from the following description of the exemplary embodiment in conjunction with the figures, in which:
Figure 1 shows an exemplary embodiment of an arrangement according to the present disclosure on the basis of an exploded view of components configured to form a pre-assembled unit for a drug delivery device.
Figure 2 shows a perspective view of an exemplary embodiment of the pre-assembled unit according to the present disclosure.
Figure 3 shows an exemplary embodiment of a module for the drug delivery device based on a simplified sectional view.
Figure 4 shows an exemplary embodiment of the drug delivery device based on a schematic longitudinal sectional view.
Figure 5 shows a simplified sectional view of a number of parts according to the present disclosure, to illustrate an embodiment of the release mechanism for releasing the releasable connection.

Like elements, elements of the same kind and identically acting elements may be provided with the same reference numerals in the figures. Additionally, the figures may be not true to scale. Rather, certain features may be depicted in an exaggerated fashion for better illustration of important principles.

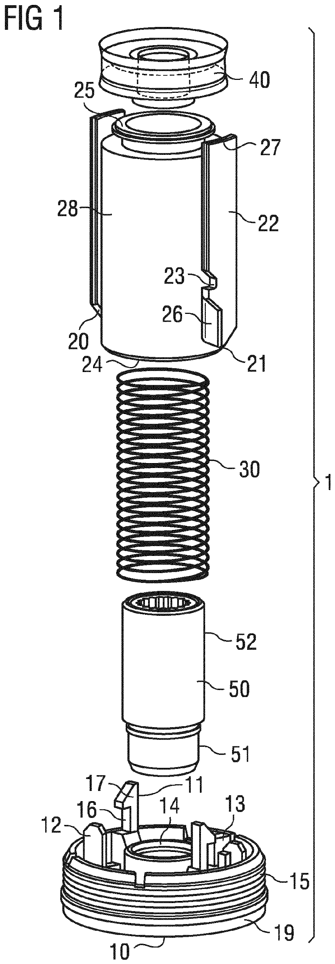

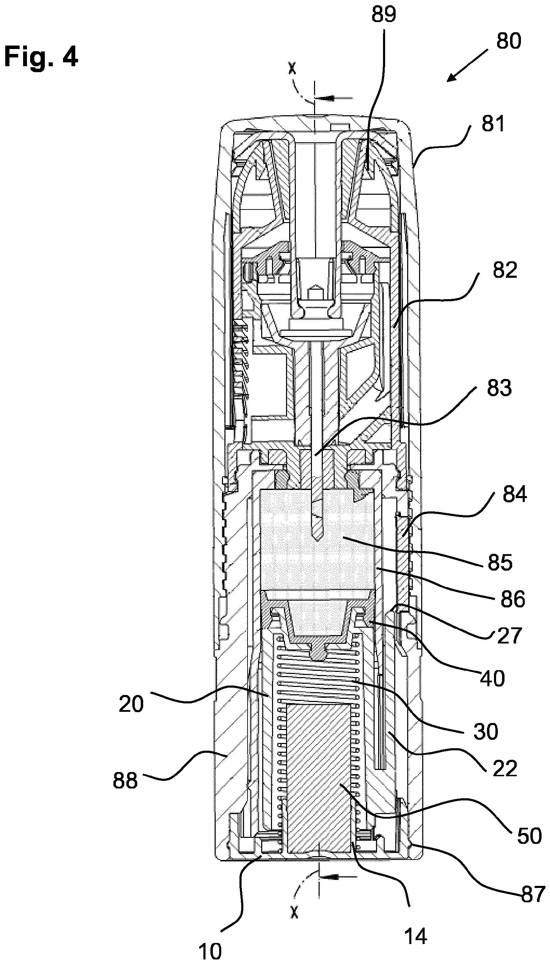

Figure 1 depicts an arrangement 1 intended for the formation of a drug delivery device 80 which is shown in Figure 4.

The arrangement comprises a support 10 and a connection part 20. The arrangement 1 further comprises a spring element 30. Furthermore, the arrangement comprises a desiccator 50. The parts of the arrangement 1 are arranged and particularly oriented along the axis x-x in the drug delivery device 80 (see Figure 4) whereas the axis may define the longitudinal axis of the drug delivery device 80. The drug delivery device may, in an exemplary embodiment, be an inhaler, particularly a dry powder inhaler. However, the disclosed concepts may also be applicable to other drug delivery devices.

The support 10 has a main body 19. The main body 19 may define a circular shape in plan view. The support 10 may form a boundary of the drug delivery device 80, e.g. its bottom. The support 10 comprises two support connection elements 11. The support connection elements 11 are arranged close to the outer edge of the support 10.

The respective support connection element 11 may protrude from the main body 19 of the support 10 in a direction along the axis, particularly towards the connection part 20. Accordingly, the respective support connection element 11 may mechanically cooperate with the connection part 20. The support connection elements 11 may comprise a shaft 16 and a projection 17 protruding from the shaft 16. The shaft 16 may be oriented axially, whereas the projection 17 may extend in the angular or tangential direction. Particularly, the connection elements are implemented hook-like. The support connection elements 11 may be implemented as snap elements or snap means. A hook-like configuration is particularly expedient for this purpose.

The respective support connection element 11 is flexible, e.g. it may be deflected, such as bent, with respect to the main body 19, expediently in an elastical or plastical fashion but without breaking.

The respective support connection element 11 comprises an oblique surface 18, particularly a surface facing towards the connection part 20. The surface may be plane. The surface has a surface normal which is oblique, particularly not perpendicular, to the axis.

It will be appreciated that the exemplary embodiment relates to two connection elements. However, there may also be just one, or more than two connection elements, such as three or more. Expediently, the number of other features or elements associated with the connection elements which are described further below may be adjusted to correspond to the number of connection elements.

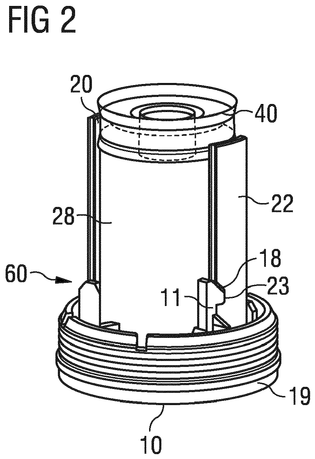

The support 10 furthermore comprises two guide features 12, one associated with each of the support connection elements 11. The respective guide feature 12 may protrude into the axial direction from the main body 19 of the support 10. The respective guide feature 12 further protrudes radially inwardly from a rim 15 which circumferentially delimits the support 10. The rim 15 may protrude axially from the main body 19. The respective guide feature 12 is of reduced length as compared to the associated support connection element 11. The respective guide feature 12 is offset, particularly angularly offset, from the associated support connection element 11. The guide feature 12 associated with one of the support connection elements 11 is arranged closer to that support connection element 11 than to the other support connection element 11. Between the support connection elements 11, a retaining space of the support 10 may be defined, wherein the desiccant 50 and the spring element 30 may be arranged, once the components of the arrangement 1 have been assembled to form the unit 60 as it is depicted in Figure 2.

The support 10 also comprises two spacers 13. The spacers 13 are arranged diametrically opposite to each other. The support 10 furthermore comprises a receiving section 14, e.g. a space radially outwardly delimited by a receiver ring protruding from the main body 19 of the support 10. The receiving section 14 may be designed to receive and retain the desiccator 50, such as by a clamping action.

The connection part 20 comprises a main body 28 and two part connection elements 21. The part connection elements 21 are elongate. The part connection elements 21 are oriented axially. The part connection elements 21 are connected to, but spaced apart from the main body 28. The main body 28 of the connection part 20 has, for example, a cylindrical shape. Of course, it may also have different shapes. The connection part 20 expediently defines a retaining space for receiving the spring element 30 and, if applicable, also the desiccator 50. Accordingly, in the assembled unit 60 said elements can be received within the connection part 20. For this purpose, the main body 28 may define an interior hollow.

The respective part connection element 21 may comprise a bar 22 and a recess 23 provided in the bar 22. The recess 23 may extend in an angular direction into the bar 22.

The support connection elements 11 and the part connection elements 21 are configured to establish a releasable connection between the support 10 and the connection part 20. The releasable connection may be a snap-fit connection. However, a releasable connection of a different type may be applied, e.g. an adhesive connection between the support 10 and the connection part 20. The shaft 16 of the respective support connection element 11 provides a spatial separation of the projection 17 from the main body 19 of the support 10. This facilitates the support connection elements 11 to be formed flexible in order to provide reliable connection elements for the releasable connection. The length of the shaft 16 may be adjusted to provide the required flexibility.

In order to establish the releasable connection of the support 10 and the connection part 20, the recesses 23 of the part connection elements 20 are brought into engagement with the projections 17 of the support connection elements 11. The recesses 23 may be matched to the projections 17 of the support connection elements 11. The recesses 23 are expediently arranged in the lower half of the connection part 20. In other words, the recesses are provided closer to that end of the connection part 20 which faces the support 10, i.e. the bottom end 24, than to the end which is remote from the support, i.e. the top end 25. The distance of the recesses 23 to the bottom end 24 is matched to the length of the shafts 16 of the support connection elements 11.

Furthermore, the part connection elements 21 are disposed diametrically opposite to one another. The same holds for the support connection elements 11. Thereby, the support or part connection elements 11 or 21 are arranged and configured such that one of those connection elements can be mapped onto the other one of those connection elements by moving the former connection element to the position of the latter connection element in the circumferential direction.

The guide features 12 and the spacers 13 are intended to guide the part connection element 21 and the connection part 20, respectively, during the connection of the support 10 and the connection part 20. The geometry of the guide features 12 and/or the spacers 13 may be rectangular, with the longer axis being oriented radially. Additionally the spacers 13 may guide the connection part 20 during the connection of the support 10 and the connection part 20 and also mechanically reinforce the support 10.

The rim 15 is provided with a projection and/or a groove embodied along the circumference of the support 10 which enables a firm and optionally a virus-tight connection to a module 70 (see Figure 4). The connection between support 10 and module 70 can, for example, be performed by ultrasonic welding.

The spring element 30 may be a metallic coil spring and is depicted with a multitude of windings. The spring element 30 is arranged between the support 10 and the connection part 20.

The desiccator 50 has a bottom portion 51 having a diameter smaller than that of a top portion 52 of the desiccator 50. The desiccator 50 may be shaped generally like a cylinder. The configuration of the bottom portion 51 is such, that it fits into the corresponding receiving section 14 of the support 10. The top portion 52 is configured to fit into an interior space delimited by the spring element 30. The outer side face of the receiving section 14 of the support 10 may simultaneously delimit the section of the support 10 which receives the spring element 30, which fits externally around or, e.g. in a friction-fit, onto the receiver ring 14. An internal groove (not shown) is arranged at the top of the interior of the connection part 20 for receiving the spring element 30 at a top end 25 of the main body 28 of the connection part 20.

In the embodiments depicted in figures 1 and 2, the bars 22 are connected to the main body 28 of the connection part 20 only in their bottom section. In other words, a connection 26 between the main body 28 and the bar 22 is provided at a position relative to the bar 22 which is closer to the bottom end of the bar 22 than to the top end. The connection may be arranged at an axial position closer to the bottom end 24 of the connection part than the recess 23. The configuration of the bars 22 is intended to provide for a mechanical stabilization of the releasable connection against external torques, e.g., being applied to the connection part 20, when the releasable connection is established (see Figure 2). The bars further comprise a rectangular cross section taken perpendicular with respect to the longitudinal axis, wherein the longer side of the rectangle is oriented tangentially.

The bars 22 may serve as a filling level indication for the filling level of drug in a reservoir 86 of the device 80, Therefore, the bars 22 are implemented to extend longitudinally as far as to the top end 25 of the main body 28. Additionally, the bars 22 aid the assembly of the drug delivery device 80 and particularly provide guiding means (see below). The space between the part connection elements 21 and the main body 28 may, in the assembled drug delivery device 80, receive the wall of the reservoir 86, such that, the main body 28 is arranged within the reservoir 86 and the connection elements are arranged outside of the reservoir 86. The connection elements 21 may, in this way be utilized for a filling level indication of the filling level of drug within the reservoir 86.

Furthermore, the connection part 20 comprises a seal member 40. The seal member 40 may be connected to the main body 28 of the connection part 20, e.g. clamped or snap-fitted to the main body 28. The seal member 40 is arranged at the top of the connection part 20. The seal member 40 may be made of an elastic material in order to effect as a sealing in the drug delivery device 80. The seal member 40 may end flush with the main body 28 of the connection part 20.

The guide features 12 are spaced along the circumference from the support connection elements 11 by a distance which corresponds to a tangential or angular dimension of the bars 22. This configuration facilitates the reception of one bar 22 between the guide feature 12 and the associated support connection element 11.

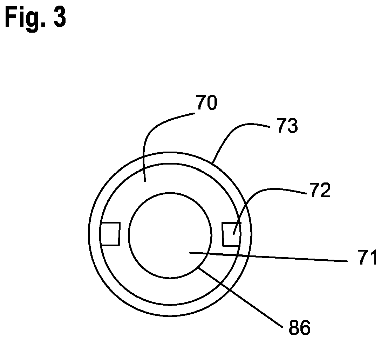

When the releasable connection is established, the arrangement 1 forms a unit 60, as depicted in Figure 2. Therein, the support 10 and the connection part 20 are releasably connected to one another with the projections 17 of the support connection elements 11 being received by the recesses 23 of the part connection elements 21. For establishing the releasable connection, the support connection elements 11 may be flexed. Furthermore the spring element 30 and the desiccator 50 are accommodated by the connection part 20 and the spring element 30 is biased, when the releasable connection is established. If the releasable connection is released, the spring element 30 may relax, thereby moving the connection part 20 away from the support 10. The support 10 forms the base of the unit 60. The support connection elements 11 provide an exposed surface 18 facing away from the bottom surface of the support 10. The exposed surface 18 is plane. The exposed surface 18 is oblique. The exposed surface 18 has a surface normal defining an acute angle with the axis. In this way, the exposed surface 18 may provide a means for releasing the releasable connection as it is exposed and may interact with a release element 72 (see Figure 3) of a module 70.

A suitable module 70 is schematically depicted in Figure 3 by means of a sectional view taken perpendicularly with respect to the axis. The module 70 comprises the release elements 72. The release elements 72 are provided at an inner surface of the module 70, e.g. a surface of a component 73 of the module 70, for example the body 88 of the device depicted in Figure 4, whereas the body 88 may define an outer housing of the drug delivery device 80, once it has been assembled. The release elements 72 are arranged diametrically opposed to each other. The release elements 72 are configured to cooperate with the exposed surfaces 18 in order to release the releasable connection when the module 70 and the unit 60 are connected for the drug delivery device 80. Thereby the tips of the release elements 72 are configured such that they protrude into the inter-space formed between the exposed surfaces 18 of the support connection element 11 and the part connection elements 21.

The module 70 may comprise a plurality of components which are pre-assembled for the drug delivery device 80. Such components may comprise the reservoir 86 for retaining the drug, a dosing mechanism suitable to extract a dose of drug from the reservoir 86 and position the dose of drug in a standby position in which the dose is ready to be inhaled during an inhalation action performed by a user of the drug delivery device 80. The reservoir 86 of the module 70 expediently has an opening 71 configured to receive the seal member 40 and optionally the main body 28 of the connection part 20. Via the opening 71, drug may be filled into the reservoir 86. The seal member 40 forms a sealed base of the filled reservoir 86 characterized by a drug-tight contact of the seal member 40 with an inner wall of the reservoir 86.

The module 70 and the unit 60 may be provided in the form of a kit for forming a ready-to-use drug delivery device 80, which may be ready for use after the reservoir 86 of the module 70 has been filled with drug and the unit 60 and the module 70 have been connected.

Figure 4 shows the drug delivery device 80, e.g., an inhaler comprising - amongst others - a cap 81, an outer cylinder 82, a dosing pin 83, a window 84, a drug 85, such as a dry powder, a reservoir 86 which contains the drug 85, a joint 87, a body 88 and/or a mouth piece 89. The drug delivery device 80 and its function is similar to the one described in WO 2009/065708 A2, in particular as far as the operation of the device is concerned.

While unscrewing the cap 81 from the body 88 and removing it from the outer cylinder 82 in this way, the dosing pin 83 is filled with a respective dose of drug 85. The suction air flow generated by a user via the mouth piece 89 then transfers the drug 85 from the dosing pin to the user. In this way, a number of doses, e.g. 40 or more, may be delivered by the drug delivery device 80. Thus, the amount of drug 85 in the reservoir 86 is continuously reduced when the drug delivery device 80 is operated repeatedly. Driven by the spring force of the spring element 30, the seal member 40 may be moved within the reservoir 86 and keep the drug 85 within the reservoir 86 under pressure. The filling level of the reservoir 86 can be observed through the window 84, through which the top section, particularly the top edge 27, of the bar 22 may be viewed which moves together with the seal member 40 in the direction towards the top end of the window while the amount of drug 85 in the reservoir 86 continuously decreases. The window 84 is dimensioned and located to cover the total travel range of the bar 22 over the whole service life of the drug delivery device 80, i.e. from a filled reservoir 86 to a reservoir 86 from which the last dose has been dispensed.

Although the connection elements 11 and 21 are not explicitly shown in Figure 4, the joint 87 of the unit 10 and the module 70 are depicted, wherein the module 70 may comprise all parts of the drug delivery device 80 except the ones present in the unit 60 and, if applicable, except the cap 81, which may be provided separately.

A method of assembling the drug delivery device 80 using the unit 60 and the module 70 as described above comprises, firstly, the step of filling of drug 85 into the reservoir 86 via the opening 71 by any suitable means. Afterwards, the module 70 and the unit 60 may be connected.

The connection of the unit 10 and the module 70 is preferably carried out by moving both parts towards each other along the axis, with respect to which both parts have been aligned previously. Thereby, at a certain point during the movement, the opening 71 of the filled reservoir 86 is closed by the seal member 40. Further movement and thereby decreasing the distance between the unit 10 and the module 70, causes the release elements 72 to come into contact with the exposed surfaces 18 of the support connection elements 11.

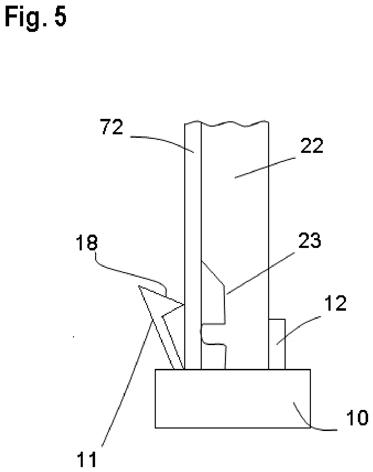

Further movement against the resilience of the support connection elements 11 causes deflection of the support connection elements 11 due to the longitudinal movement of the release elements 72 being converted into a tangential or angular movement of the projection 17 by means of the release elements 72 sliding along the exposed and expediently oblique surface. The tangential or angular deflection causes bending of the support connection elements 11, thereby separating the projections 17 of the support connection elements 11 from the recesses 23 of the part connection elements 21 and releasing the releasable connection. Figure 5 shows a simplified representation of components involved in releasing the releasable connection. The part connection elements 21, particularly the bars 23, and the release elements 72 may, in cooperation, rotationally lock the module 70 and the unit 60 to each other, such that relative rotation therebetween is prevented or inhibited. In this way, reliable cooperation of the release elements 72 and the support connection elements 11 may be ensured to release the releasable connection.

As a consequence, after the releasable connection was released, the connection part 20 becomes moveable with respect to the support 10, since the spring element 30 partly relaxes. By means of the spring element 30, the seal member 40 may be moved into and/or kept in contact with the drug 85 in the reservoir 86 and consequently, a mechanical pressure is exerted on the drug 85. To this effect, the pressure effects the trailing or movement of the seal member 40 which forms a movable base of the reservoir 86.

Although it is described above that the support connection element is flexible, the disclosed concept would also work with a flexible part connection element.

The term "drug" as used herein may mean a pharmaceutical formulation containing at least one pharmaceutically active compound, for example for the treatment of obstructive airway or lung diseases such as asthma or chronic obstructive pulmonary disease (COPD), local respiratory tract oedema, inflammation, viral, bacterial, mycotic or other infection, allergies, diabetes mellitus.

The active pharmaceutical compound is preferably selected from the group consisting of active pharmaceutical compounds suitable for inhalation, preferably antiallergenic, antihistamine, anti-inflammatory, antitussive agents, bronchodilators, anticholinergic drugs, and combinations thereof.

The active pharmaceutical compound may for example be chosen from:
an insulin such as human insulin, e.g. a recombinant human insulin, or a human insulin analogue or derivative, a glucagon-like peptide (GLP-1) or an analogue or derivative thereof, or exendin-3 or exendin-4 or an analogue or derivative of exendin-3 or exendin-4;
an adrenergic agent such as a short acting β2-agonists (e.g. Salbutamol, Albuterol, Levosalbutamol, Fenoterol, Terbutaline, Pirbuterol, Procaterol, Bitolterol, Rimiterol, Carbuterol, Tulobuterol, Reproterol), a long acting β2-agonist (LABA, e.g. Arformoterol, Bambuterol, Clenbuterol, Formoterol, Salmeterol), an ultra LABA (e.g. Indacaterol) or another adrenergic agent (e.g. Epinephrine, Hexoprenaline, Isoprenaline (Isoproterenol), Orciprenaline (Metaproterenol));
a glucocorticoid (e.g. Beclometasone, Budesonide, Ciclesonide, Fluticasone, Mometasone, Flunisolide, Betamethasone, Triamcinolone);
an anticholinergic agent or muscarinic antagonist (e.g. Ipratropium bromide, Oxitropium bromide, Tiotropium bromide);
a mast cell stabilizer (e.g. Cromoglicate, Nedocromil);
a xanthine derivative (e.g. Doxofylline, Enprofylline, Theobromine, Theophylline, Aminophylline, Choline theophyllinate);
an eicosanoid inhibitor, such as a leukotriene antagonist (e.g. Montelukast, Pranlukast, Zafirlukast), a lipoxygenase inhibitor (e.g. Zileuton) or a thromboxane receptor antagonist (e.g. Ramatroban, Seratrodast);
a phosphodiesterase type-4 inhibitor (e.g. Roflumilast);
an antihistamine (e.g. Loratadine, Desloratadine, Cetirizen, Levocetirizine, Fexofenadine);
an allergen immunotherapy (e.g. Omalizumab);
a mucolytic (e.g. Carbocisteine, Erdosteine, Mecysteine);
an antibiotic or antimycotic;
or a combination of any two, three or more of the above-mentioned compound classes or compounds (e.g. Budesonide/Formoterol, Fluticasone/Salmeterol, Ipratropium bromide/Salbutamol, Mometasone/Formoterol);
or a pharmaceutically acceptable salt or solvate or esters of any of the above named compounds.

Pharmaceutically acceptable salts are for example acid addition salts and basic salts. Acid addition salts are e.g. a chloride, bromide, iodide, nitrate, carbonate, sulfate, methylsulfate, phosphate, acetate, benzoate, benzenesulfonate, fumarate, malonate, tartrate, succinate, citrate, lactate, gluconate, glutamate, edetate, mesylate, pamoate, pantothenate or a hydroxy-naphthoate salt. Basic salts are for example salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology. Pharmaceutically acceptable ester may for example be acetates, propionates, phosphates, succinates or etabonates.

Pharmaceutically acceptable solvates are for example hydrates.

The scope of protection of the invention is not limited to the examples given hereinabove. The invention is embodied in each novel characteristic and each combination of characteristics, which particularly includes every combination of any features which are stated in the claims, even if this feature or this combination of features is not explicitly stated in the examples.

### Reference numerals

- 1: Arrangement
- 10: Support
- 11: Support connection element
- 12: Guide feature
- 13: Spacer
- 14: Receiving section
- 15: Rim
- 16: Shaft
- 17: Projection
- 18: Exposed surface
- 19: Main body
- 20: Connection part
- 21: Part connection element
- 22: Bar
- 23: Recess
- 24: Bottom end
- 25: Top end
- 26: Connection
- 27: Top edge
- 28: Main body
- 30: Spring element
- 40: Seal member
- 50: Desiccator
- 51: Bottom portion
- 52: Top portion
- 60: Unit
- 70: Module
- 71: Opening
- 72: Release element
- 73: Component of module
- 80: Drug delivery device
- 81: Cap
- 82: Outer cylinder
- 83: Dosing pin
- 84: Window
- 85: Drug
- 86: Reservoir
- 87: Joint
- 88: Body
- 89: Mouth piece

- x-x: axis

## Claims

1. An inhaler (80) for dispensing a drug (85), comprising an arrangement (1) for the inhaler (80), the arrangement comprising
a support (10),
a connection part (20), and
a spring element (30), wherein
- the spring element (30) is arranged between the support (10) and the connection part (20),
- the support (10) comprises a support connection element (11) and the connection part (20) comprises a part connection element (21), wherein these connection elements are configured to establish a releasable connection to releasably connect the support (10) and the connection part (20), and
the inhaler further comprising a module (70) which is connected to the arrangement (1), the module (70) comprising a reservoir (86) which contains the drug (85), wherein the releasable connection between the support (10) and the connection part (20) is not established, and the spring element (30) is arranged to move the connection part (20) away from the support (10) when the inhaler (80) is operated to dispense a dose of the drug (85) such that the drug is kept under pressure by the connection part (20).

2. The inhaler according to claim 1, wherein the releasable connection is a snap-fit connection.

3. The inhaler according to claim 1 or 2, wherein the support connection element (11) and the part connection element (21) are oriented along a common axis.

4. The inhaler according to claim 3, wherein the arrangement (1) is configured such that, when the releasable connection is established, one of the support connection element (11) and the part connection element (21) has an exposed surface (18) with a surface normal defining an acute angle with the axis.

5. The inhaler according to at least one of the previous claims, wherein the part connection element (21) comprises an axially oriented bar (22), which is spaced apart from and connected to a main body (28) of the connection part (20).

6. The inhaler according to at least one of the previous claims, wherein, in the arrangement (1), the support connection element (11) and the part connection element (21) are arranged and configured such that, when the releasable connection is not established, and the distance between the support connection element (11) and the part connection element (21) is decreased by a relative movement between the support (10) and the connection part (20), the spring element (30) is biased and the support connection element (11) and the part connection element (21) engage one another, thereby releasably connecting the support (10) and the connection part (20) and preventing relative movement between the support connection element (11) and the part connection element (21), which would increase the distance between the support connection element (11) and the part connection element (21).

7. The inhaler according to at least one of the previous claims, wherein the arrangement (1) is configured such that, when the releasable connection is established, the spring element (30) is biased and disengagement of the support connection element (11) and the part connection element (21) is prevented by the spring force of the biased spring element (30) which keeps the support connection element (11) and the part connection element (21) in abutment.

8. The inhaler according to at least one of the previous claims, wherein the support (10) comprises a plurality of support connection elements (11) and the connection part (20) comprises a plurality of part connection elements (21), the part connection elements (21) being configured, in each case, to mate with a respective one of the support connection elements (11), and wherein the support connection elements (11) and the part connection elements (21) are uniformly distributed on the support (10) and the connection part (20), respectively.

9. The inhaler (80) according to any one of the preceding claims, wherein the connection part (20) comprises a seal member (40) suitable to form a drug-tight seal in cooperation with a wall of the reservoir (86).

10. The inhaler (80) according to claim 9, wherein the reservoir (86) contains a plurality of doses of the drug (85), and wherein the seal member forms the movable sealing base of the reservoir.

11. A method of assembling an inhaler (80) comprising the following steps:
- providing a unit (60), the unit (60) comprising a support (10) and a connection part (20) which are releasably connected to each other;
- providing a module (70), the module (70) comprising a reservoir (86) and an opening (71) communicating with the reservoir (86);
- filling drug (85) into the reservoir (86) via the opening (71); and
- connecting the unit (60) and the module (70) with each other, closing the opening (71) of the filled reservoir (86) with the connection part (20) and releasing the releasable connection such that the connection part (20) is movable with respect to the support (10) and such that the drug is kept under pressure by the connection part (20).

12. The method according to claim 11, wherein the module (70) comprises a release element (72) configured to cooperate with at least one of the part connection element (21) and the support connection element (11) to release the releasable connection during relative movement of unit (60) and module (70) to establish the connection of the unit (60) and the module (70).

13. The method according to claim 12, wherein one of the part connection element (21) and the support connection element (11) is flexible, and wherein the assembling is carried out by moving the unit (60) and the module (70) axially towards one another, thereby bringing the release element (72) in cooperation with an exposed surface (18) of one of the part connection element (21) and the support connection element (11), further movement causing a deflection of the flexible one of the support connection element (11) and the part connection element (21), thereby disengaging the part connection element (21) and the support connection element (11) and releasing the releasable connection, and wherein the flexible one of the support connection element (11) and the part connection element (21) remains deflected, after the releasable connection has been released.

14. A kit comprising:
a) a unit (60) with an arrangement (1) for an inhaler (80), the arrangement comprising
a support (10),
a connection part (20),
and a spring element (30),
wherein the spring element (30) is arranged between the support (10) and the connection part (20), and wherein the support (10) comprises a support connection element (11) and the connection part (20) comprises a part connection element (21), wherein these connection elements are configured to establish a releasable connection to releasably connect the support (10) and the connection part (20), wherein the releasable connection is established; and
b) a module (70) connectable to the unit (60), wherein the module (70) comprises:
at least one release element (72) configured to cooperate with at least one of the support connection element (11) and the part connection element (21) to release the releasable connection when the unit (60) and the module (70) are connected to one another, and
a reservoir (86) configured to retain a drug to be dispensed from the inhaler, wherein the reservoir is arranged at an open end of the module (70), and wherein the open end and the reservoir are configured to receive the connection part, wherein the kit is configured such that the drug is kept under pressure by the connection part (20) when the releasable connection is released.

## Patentansprüche

1. Inhalator (80) zur Abgabe eines Arzneimittels (85), umfassend eine Anordnung (1) für den Inhalator (80), wobei die Anordnung das Folgende umfasst einen Träger (10),
ein Verbindungsteil (20) und
ein Federelement (30), wobei
- das Federelement (30) zwischen dem Träger (10) und dem Verbindungsteil (20) angeordnet ist,
- der Träger (10) ein Trägerverbindungselement (11) umfasst und das Verbindungsteil (20) ein Teilverbindungselement (21) umfasst, wobei die Verbindungselemente zur Herstellung einer lösbaren Verbindung ausgelegt sind, um den Träger (10) und das Verbindungsteil (20) lösbar miteinander zu verbinden,
und
wobei der Inhalator ferner ein Modul (70) umfasst, das mit der Anordnung (1) verbunden ist, wobei das Modul (70) ein Reservoir (86) umfasst, welches das Arzneimittel (85) enthält, wobei die lösbare Verbindung zwischen dem Träger (10) und dem Verbindungsteil (20) nicht hergestellt ist, und das Federelement (30) dazu angeordnet ist, das Verbindungsteil (20) vom Träger (10) weg zu bewegen, wenn der Inhalator (80) zur Ausgabe einer Dosis des Arzneimittels (85) betätigt wird, so dass das Arzneimittel vom Verbindungsteil (20) unter Druck gehalten wird.

2. Inhalator nach Anspruch 1, wobei die lösbare Verbindung eine Schnappverbindung ist.

3. Inhalator nach Anspruch 1 oder 2, wobei das Trägerverbindungselement (11) und das Teilverbindungselement (21) entlang einer gemeinsamen Achse orientiert sind.

4. Inhalator nach Anspruch 3, wobei die Anordnung (1) derart ausgelegt ist, dass, wenn die lösbare Verbindung hergestellt ist, das Trägerverbindungselement (11) oder das Teilverbindungselement (21) eine freiliegende Oberfläche (18) aufweist, wobei eine Oberflächennormale einen spitzen Winkel mit der Achse definiert.

5. Inhalator nach mindestens einem der vorhergehenden Ansprüche, wobei das Teilverbindungselement (21) einen axial orientierten Stab (22) umfasst, der im Abstand von einem Hauptkörper (28) des Verbindungsteils (20) angeordnet und damit verbunden ist.

6. Inhalator nach mindestens einem der vorhergehenden Ansprüche, wobei in der Anordnung (1) das Trägerverbindungselement (11) und das Teilverbindungselement (21) derart angeordnet und ausgelegt sind, dass, wenn die lösbare Verbindung nicht hergestellt ist, und der Abstand zwischen dem Trägerverbindungselement (11) und dem Teilverbindungselement (21) durch eine relative Bewegung zwischen dem Träger (10) und dem Verbindungsteil (20) verkleinert wird, das Federelement (30) vorgespannt wird und das Trägerverbindungselement (11) und das Teilverbindungselement (21) miteinander in Eingriff kommen, wodurch der Träger (10) und das Verbindungsteil (20) lösbar miteinander verbunden werden und eine relative Bewegung des Trägerverbindungselements (11) und des Teilverbindungselements (21), die den Abstand zwischen dem Trägerverbindungselement (11) und dem Teilverbindungselement (21) vergrößern würde, verhindert wird.

7. Inhalator nach mindestens einem der vorhergehenden Ansprüche, wobei die Anordnung (1) derart ausgelegt ist, dass, wenn die lösbare Verbindung hergestellt ist, das Federelement (30) vorgespannt wird und Lösen des Eingriffs des Trägerverbindungselements (11) und des Teilverbindungselements (21) durch die Federkraft des vorgespannten Federelements (30), die das Trägerverbindungselement (11) und das Teilverbindungselement (21) in Anlage hält, verhindert wird.

8. Inhalator nach mindestens einem der vorhergehenden Ansprüche, wobei der Träger (10) eine Vielzahl von Trägerverbindungselementen (11) umfasst und das Verbindungsteil (20) eine Vielzahl von Teilverbindungselementen (21) umfasst, wobei die Teilverbindungselemente (21) jeweils dazu ausgelegt sind, mit einem jeweiligen der Trägerverbindungselemente (11) zusammenzupassen, und wobei die Trägerverbindungselemente (11) und die Teilverbindungselemente (21) gleichmäßig auf dem Träger (10) bzw. auf dem Verbindungsteil (20) verteilt sind.

9. Inhalator (80) nach einem der vorhergehenden Ansprüche, wobei das Verbindungsteil (20) ein Dichtungselement (40) umfasst, das zur Bildung einer arzneimitteldichten Abdichtung in Zusammenarbeit mit einer Wand des Reservoirs (86) geeignet ist.

10. Inhalator (80) nach Anspruch 9, wobei das Reservoir (86) eine Vielzahl von Dosen des Arzneimittels (85) enthält, und wobei das Dichtungselement den beweglichen dichtenden Boden des Reservoirs bildet.

11. Verfahren zum Zusammenbau eines Inhalators (80), umfassend die folgenden Schritte:
- Bereitstellen einer Einheit (60), wobei die Einheit (60) einen Träger (10) und ein Verbindungsteil (20) umfasst, die lösbar miteinander verbunden sind;
- Bereitstellen eines Moduls (70), wobei das Modul (70) ein Reservoir (86) und eine mit dem Reservoir (86) in Verbindung stehende Öffnung (71) umfasst;
- Einfüllen des Arzneimittels (85) in das Reservoir (86) durch die Öffnung (71); und
- Verbinden der Einheit (60) und des Moduls (70) miteinander, Schließen der Öffnung (71) des gefüllten Reservoirs (86) mit dem Verbindungsteil (20) und Lösen der lösbaren Verbindung, so dass das Verbindungsteil (20) bezüglich des Trägers (10) beweglich ist und so dass das Arzneimittel vom Verbindungsteil (20) unter Druck gehalten wird.

12. Verfahren nach Anspruch 11, wobei das Modul (70) ein Freigabeelement (72) umfasst, das zur Zusammenarbeit mit mindestens dem Teilverbindungselement (21) oder dem Trägerverbindungselement (11) ausgelegt ist, um die lösbare Verbindung während einer relativen Bewegung der Einheit (60) und des Moduls (70) zu lösen, um die Verbindung der Einheit (60) und des Moduls (70) herzustellen.

13. Verfahren nach Anspruch 12, wobei das Teilverbindungselement (21) oder das Trägerverbindungselement (11) flexibel ist, und wobei der Zusammenbau dadurch erfolgt, dass die Einheit (60) und das Modul (70) axial aufeinander zu bewegt werden, wodurch das Freigabeelement (72) in Zusammenarbeit mit einer freiliegenden Fläche (18) des Teilverbindungselements (21) oder des Trägerverbindungselements (11) gebracht wird, wobei eine weitere Bewegung eine Ablenkung des flexiblen Elements des Trägerverbindungselements (11) und des Teilverbindungselements (21) verursacht, wodurch der Eingriff des Teilverbindungselements (21) und des Trägerverbindungselements (11) gelöst wird und die lösbare Verbindung gelöst wird, und wobei das flexible des Trägerverbindungselements (11) und des Teilverbindungselements (21) abgelenkt bleibt, nachdem die lösbare Verbindung gelöst wurde.

14. Bausatz, umfassend:
a) eine Einheit (60) mit einer Anordnung (1) für einen Inhalator (80), wobei die Anordnung das Folgende umfasst
einen Träger (10),
ein Verbindungsteil (20),
und ein Federelement (30),
wobei das Federelement (30) zwischen dem Träger (10) und dem Verbindungsteil (20) angeordnet ist, und wobei der Träger (10) ein Trägerverbindungselement (11) umfasst und das Verbindungsteil (20) ein Teilverbindungselement (21) umfasst, wobei diese Verbindungselemente zur Herstellung einer lösbaren Verbindung ausgelegt sind, um den Träger (10) und das Verbindungsteil (20) lösbar miteinander zu verbinden, wobei die lösbare Verbindung hergestellt ist; und
b) ein Modul (70), das mit der Einheit (60) verbindbar ist, wobei das Modul (70) das Folgende umfasst:
mindestens ein Freigabeelement (72), das zur Zusammenarbeit mit mindestens dem Teilverbindungselement (21) oder dem Trägerverbindungselement (11) ausgelegt ist, um die lösbare Verbindung zu lösen, wenn die Einheit (60) und das Modul (70) miteinander verbunden werden, und
ein Reservoir (86), das zum Aufbewahren eines Arzneimittels, das aus dem Inhalator abgegeben werden soll, ausgelegt ist, wobei das Reservoir an einem offenen Ende des Moduls (70) angeordnet ist und wobei das offene Ende und das Reservoir zur Aufnahme des Verbindungsteils ausgelegt sind, wobei der Bausatz derart ausgelegt ist, dass das Arzneimittel vom Verbindungsteil (20) unter Druck gehalten wird, wenn die lösbare Verbindung gelöst ist.

## Revendications

1. Inhalateur (80) pour administrer un médicament (85), comprenant un agencement (1) pour l'inhalateur (80), l'agencement comprenant :
un support (10),
une pièce de raccordement (20), et
un élément de ressort (30), dans lequel :
- l'élément de ressort (30) est disposé entre le support (10) et la pièce de raccordement (20),
- le support (10) comprend un élément de raccordement (11) du support et la pièce de raccordement (20) comprend un élément de raccordement (21) de la pièce,
dans lequel ces éléments de raccordement sont configurés pour établir un raccordement amovible afin de raccorder de façon amovible le support (10) et la pièce de raccordement (20), et
l'inhalateur comprenant en outre un module (70) qui est raccordé à l'agencement (1), le module (70) comprenant un réservoir (86) qui contient le médicament (85),
dans lequel le raccordement amovible entre le support (10) et la pièce de raccordement (20) n'est pas établi et l'élément de ressort (30) est agencé pour éloigner la pièce de raccordement (20) du support (10) quand l'inhalateur (80) est utilisé pour administrer une dose du médicament (85) de telle sorte que le médicament soit maintenu sous pression par la pièce de raccordement (20).

2. Inhalateur selon la revendication 1, dans lequel le raccordement amovible est un raccord encliquetable.

3. Inhalateur selon la revendication 1 ou 2, dans lequel l'élément de raccordement (11) du support et l'élément de raccordement (21) de la pièce sont orientés suivant un axe commun.

4. Inhalateur selon la revendication 3, dans lequel l'agencement (1) est configuré de telle sorte que, quand le raccordement amovible est établi, soit l'élément de raccordement (11) du support soit l'élément de raccordement (21) de la pièce comporte une surface (18) apparente avec une surface perpendiculaire faisant un angle aigu avec l'axe.

5. Inhalateur selon au moins une des revendications précédentes, dans lequel l'élément de raccordement (21) de la pièce comprend une barre (22) orientée axialement, qui est espacée du corps principal (28) de la pièce de raccordement (20) et raccordée à celui-ci.

6. Inhalateur selon au moins une des revendications précédentes, dans lequel, dans l'agencement (1), l'élément de raccordement (11) du support et l'élément de raccordement (21) de la pièce sont disposés et configurés de telle sorte que, quand le raccordement amovible n'est pas établi et que la distance entre l'élément de raccordement (11) du support et l'élément de raccordement (21) de la pièce est diminuée par un mouvement relatif entre le support (10) et la pièce de raccordement (20), l'élément de ressort (30) est sollicité et l'élément de raccordement (11) du support et l'élément de raccordement (21) de la pièce s'enclenchent l'un avec l'autre, raccordant de ce fait de façon amovible le support (10) et la pièce de raccordement (20) et empêchant un mouvement relatif entre l'élément de raccordement (11) du support et l'élément de raccordement (21) de la pièce, qui augmenterait la distance entre l'élément de raccordement (11) du support et l'élément de raccordement (21) de la pièce.

7. Inhalateur selon au moins une des revendications précédentes, dans lequel l'agencement (1) est configuré de telle sorte que, quand le raccordement amovible est établi, l'élément de ressort (30) est sollicité et la désolidarisation de l'élément de raccordement (11) du support et de l'élément de raccordement (21) de la pièce est empêchée par la force élastique de l'élément de ressort (30) sollicité qui maintient en butée l'élément de raccordement (11) du support et l'élément de raccordement (21) de la pièce.

8. Inhalateur selon au moins une des revendications précédentes, dans lequel le support (10) comprend une pluralité d'éléments de raccordement (11) du support et la pièce de raccordement (20) comprend une pluralité d'éléments de raccordement (21) de la pièce, les éléments de raccordement (21) de la pièce étant configurés, dans chaque cas, pour s'accoupler avec l'un des éléments de raccordement (11) correspondant du support, et dans lequel les éléments de raccordement (11) du support et les éléments de raccordement (21) de la pièce sont uniformément répartis, respectivement, sur le support (10) et la pièce de raccordement (20).

9. Inhalateur (80) selon l'une quelconque des revendications précédentes, dans lequel la pièce de raccordement (20) comprend un élément d'étanchéité (40) convenant à constituer, en coopération avec une paroi du réservoir (86), un joint étanche au médicament.

10. Inhalateur (80) selon la revendication 9, dans lequel le réservoir (86) contient une pluralité de doses du médicament (85), et dans lequel l'élément d'étanchéité constitue la base d'étanchéité mobile du réservoir.

11. Procédé d'assemblage d'un inhalateur (80), comprenant les étapes consistant à :
- faire appel à une unité (60), l'unité (60) comprenant un support (10) et une pièce de raccordement (20) qui sont raccordées l'un à l'autre de façon amovible ;
- faire appel à un module (70), le module (70) comprenant un réservoir (86) et une ouverture (71) communiquant avec le réservoir (86) ;
- remplir de médicament (85) le réservoir (86) par l'ouverture (71) ; et
- raccorder l'une à l'autre l'unité (60) et le module (70), fermer avec la pièce de raccordement (20) l'ouverture (71) du réservoir (86) rempli et libérer le raccordement amovible de telle sorte que la pièce de raccordement (20) soit mobile par rapport au support (10) et de telle sorte que le médicament soit maintenu sous pression par la pièce de raccordement (20).

12. Procédé selon la revendication 11, dans lequel le module (70) comprend un élément de débrayage (72) configuré pour coopérer avec au moins d'un élément de raccordement (21) de la pièce et l'élément de raccordement (11) du support afin de libérer le raccordement amovible pendant le mouvement relatif de l'unité (60) et du module (70) afin d'établir le raccordement de l'unité (60) et du module (70).

13. Procédé selon la revendication 12, dans lequel soit l'élément de raccordement (21) de la pièce soit l'élément de raccordement (11) du support est flexible, et dans lequel on effectue l'assemblage en déplaçant axialement l'une vers l'autre l'unité (60) et le module (70), amenant de ce fait l'élément de débrayage (72) à coopérer avec une surface (18) apparente de soit l'élément de raccordement (21) de la pièce soit l'élément de raccordement (11) du support, un déplacement ultérieur provoquant un fléchissement de celui de l'élément de raccordement (11) du support et de l'élément de raccordement (21) de la pièce qui est flexible, désolidarisant de ce fait l'élément de raccordement (21) de la pièce et l'élément de raccordement (11) du support est flexible et libérant le raccordement amovible, et dans lequel celui de l'élément de raccordement (11) du support et de l'élément de raccordement (21) de la pièce qui est flexible reste fléchi après que le raccordement amovible a été libéré.

14. Nécessaire comprenant :
a) une unité (60) ayant un agencement (1) pour un inhalateur (80), l'agencement comprenant :
un support (10),
une pièce de raccordement (20), et
un élément de ressort (30),
dans lequel l'élément de ressort (30) est disposé entre le support (10) et la pièce de raccordement (20), et dans lequel le support (10) comprend un élément de raccordement (11) du support et la pièce de raccordement (20) comprend un élément de raccordement (21) de la pièce, dans lequel ces éléments de raccordement sont configurés pour établir un raccordement amovible afin de raccorder de façon amovible le support (10) et la pièce de raccordement (20), dans lequel le raccordement amovible est établi ; et
b) un module (70) pouvant être raccordé à l'unité (60), dans lequel le module (70) comprend :
au moins un élément de débrayage (72) configuré pour coopérer avec au moins d'un élément de raccordement (11) du support et l'élément de raccordement (21) de la pièce afin de libérer le raccordement amovible quand l'unité (60) et le module (70) sont raccordés l'une à l'autre, et
un réservoir (86) configuré pour retenir un médicament à administrer à partir de l'inhalateur, dans lequel le réservoir est disposé à une extrémité ouverte du module (70), et
dans lequel l'extrémité ouverte et le réservoir sont configurés pour recevoir la pièce de raccordement, dans lequel le nécessaire est configuré de telle sorte que le médicament soit maintenu sous pression par la pièce de raccordement (20) quand le raccordement amovible est libéré.
